# EUROPEAN PATENT SPECIFICATION

(11) **EP 3 772 919 B1**
(45) Date of publication and mention of the grant of the patent: **08.01.2025**
(21) Application number: 19784573.8
(22) Date of filing: 10.04.2019
(51) Int. Cl.: A01K 13/00

(54) **ANIMAL TREATMENT BLOCK**
TIERBEHANDLUNGSBLOCK
BLOC DE TRAITEMENT D'ANIMAL

(30) Priority: 12.04.2018 AU 2018901232
(43) Date of publication of application: 17.02.2021
(73) Proprietor: Bradshaw, Glenn Andrew, Tarzali QLD 4885 (AU)
(72) Inventor: Bradshaw, Glenn Andrew, Tarzali QLD 4885 (AU)
(74) Representative: Gallafent, Richard John
(86) International application number: PCT/AU2019/050315
(87) International publication number: WO 2019/195883

(56) References cited:
- WO-A1-2015/026906
- CA-A1- 2 104 990
- GB-A- 779 581
- US-A- 2 581 028
- US-A- 3 826 232
- US-A- 3 826 232
- US-A- 6 035 807
- US-A1- 2003 150 467
- US-A1- 2003 150 467
- US-A1- 2004 202 690
- US-A1- 2010 125 097

## Description

### FIELD OF THE INVENTION

The present invention relates to an animal treatment block for treatment of animals against the action of biting insects.

### BACKGROUND OF THE INVENTION

Cattle, in particular, tend to have problems with biting insects, such as biting flies. Biting flies such as *haematobia irritans*, known as buffalo fly or horn fly, are a serious and significant problem for cattle, and hence for cattle producers. More than simply being a nuisance, the buffalo flies and other biting insects, such as sucking lice *linognathus,* or biting lice *bovicola bovis,* can cause significant stress and discomfort to the cattle. The cattle try to rid themselves of the biting insects around their face and body, causing them to be restless and irritable. The constant moving and distress inevitably lead to the animal to lose weight and condition. Where the cattle are being raised for meat, the low sale weight is a negative consequence of the insects continually bothering the animal and the restless behaviour. Further, damage to the hides can be caused by the action of the biting insects and the cattle trying to relieve themselves of the irritation. Damaged hides are undesirable as this reduces the potential sale value to the cattle producer.

Parasites, disease and general malaise may be a further result of the cattle being continually pestered by the flies and other biting insects. To address the significant problem these animals have long been treated with insecticides to try to keep the animals healthy and happy. Manual application of suitable chemicals to kill the insects is the usual way, through spray, dips or injections. Use of an ear tag is also known, containing active ingredient to treat the animal; this works but is costly. Application of each of these direct methods tends to be extremely time consuming and labour intensive. With a manual application every animal needs to be sufficiently treated through direct contact, to act against the troublesome insects. Where the animals roam a large area the animals would need to be rounded up and treated before returning to grazing. It is not uncommon for animals to spread over a very wide area of bushland, whereby the cost and inconvenience of making these treatments means that they simply do not occur and the animals must suffer the insects.

The present solution to the problem is to anticipate popular gathering points for the cattle, such as next to water, and position treatment points for the animals at these points. These hanging apparatus treatments can take the form of a diesel or white oil soaked thick hessian rope, or sausage shaped piece of material, hung between two points. Other liquid oil carriers are also known. The apparatus is slung at a height slightly below that of the back of a typical animal walking underneath so that it will make contact. Where the animal is looking to scratch their back, use of the slung material acts to also apply the treatment, and provide scratching relief.

In one known application method using diesel, the diesel acts as the medium to carry and apply the insecticide to the animal, when it contacts the hanging material. The contact may be along the back, neck, side or face of the animal, applying the active ingredient mixed with the diesel as the animal scratches and rubs. The diesel is useful as a medium but is also a very unpleasant product to use, not the most desirable material to be handled by the person, or entering the environment. More significantly, diesel is a liquid hydrocarbon that it is undesirable to have dripping on the ground, particularly when mixed with an insecticide. Environmental protections at industrial and mine sites generally restrict such materials being allowed to be spilled or drip on the ground due to the potential environmental impact. It is desirable therefore to better control materials, and application of the insecticide to animals, so that these do not drip uncontrolled into the ground.

A great many of these conventional hanging applicators must be set up to cover an area. The person who is setting up the hanging apparatus will inevitably come in to direct contact with the diesel or other carrier and insecticides, potentially risking their own health as a consequence. Animals should have the insecticide applied every few days for continued action against the biting insects. The current method of treatment must be recharged every two weeks, or so, if the treatment of the animals against biting insects is to continue. The reason for this is a combination of the carrier dripping as it is liquid, as well as it being used up through the animal contact, drying out in the sun, or possibly displaced in a rain event. Two weeks is a very short time, meaning that there is a risk that the task will not be performed regularly enough, the flies will not be killed and the cattle will be pestered by the biting flies.

Clearly, as a liquid product, the diesel or other liquid carrier, is vulnerable to this rapid degradation from the apparatus. The release of the diesel or other liquid carrier is utterly uncontrolled, at the time of charging the apparatus the excess will simply drip from the hessian, and over time as the volume of liquid carrier decreases the remainder may dry out in the heat and wind. The liquid carrier drips when first charged from the hessian and can take the active ingredient into the ground, wasting the active ingredient, at the start of use. In heavy rain, the product can be washed from the soaked material, displacing the hydrocarbon with water.

It can be, therefore, very costly for the property owner to need to keep replenishing the diesel and active ingredients. Shelters may be built over conventional back rubbers so that these are protected to some extent from excess heat, drying out or rain. The building of these shelters may be inconvenient, and takes time and resources. The chemicals used to give relief to the cattle are generally considered safe to use as needed on the animal, but are harsh chemicals considered "toxic" as chemicals themselves. It is most advisable not to place the diesel soaked applicators anywhere near water for risk of the adverse consequences to the environment and wildlife. However, this is where it is desirable to place the devices, in the shade and near water where the animals congregate. It would, therefore, be most desirable to be able to relieve the cattle of the biting flies and other insects, in a controlled fashion, without the risk of contamination to the ground or water. Further, it would be highly beneficial for the cattle producer to be able to install a treatment that will then go on being effective against the biting insects for many weeks and months.

A pest control package comprising a tubular receptacle and a pest control composition in solid stable tubular stick form is known from US3826232 A.

The inventor has developed a significant improvement over the art, which is likely to be universally adopted once known, and which is defined in the appended claims. The inventor has developed, in a favoured form, a wax cylinder block according to claim 1 for a controlled release and continual application of insecticide for animals for 12 or up to 20 weeks. The wax block with reinforcing fibres is safer and easier to handle, keeping the active ingredients in solid form, protected against accidental spillage. The cylinder block can be simply installed, by threading onto a rope or chain between two points, without the need to contact the active ingredients. Once in the field the especially designed reinforced wax structure resists degradation, and will not drip or wash away. Instead the wax cylinder block is sturdy and resilient to the harsh conditions in the bush, enabling treatment to each animal as it contacts and rolls over its back, and face, rubbing to remove the itch of the biting insects and by doing so transferring some of the wax medium and active ingredient to their body.

In this way a single application of treatment blocks to the regular cattle spots can enable treatment of the animals for 12 to 20 weeks, instead of for only around two weeks, reducing the workload in this regard. Most importantly, by making the application of the treatments for these cattle easier and more useful in the longer term means that the cattle are less bothered by the insects. Being less bothered by the insects means that the cattle are less restless, less irritated and will present with a better sale weight than cattle that are constantly moving to try to get rid of the flies.

Ultimately, happy cattle is a positive result for the animal and the farmer. The subject invention can be used with beef cattle at any stage of development, calves through to a mature animal and can be adapted for use with any livestock, including horses, goats and deer. The teachings of the present disclosure can be adapted for use for any suitable animal and animal treatment.

The present invention resolves many of the issues of the prior art and can also be provided in a more environmentally friendly product form, through use of an organic essential oil based product in an animal treatment block according to claim 1. Having an organic option for farmers wishing to avoid the use of some of the harsh insecticides, and reduce biting insects for the cattle, this is a further strong advantage to the invention that is likely to prove popular for organic farmers and the hobby farmers alike. Combinations of active ingredient with essential oil based products can be used too, to provide a wide range of suitable treatment blocks for the needs of different animals and environments.

Overall the inventor has developed a very useful and beneficial invention that will ultimately improve profitability for cattle farmer, while improving the health and comfort of the animals. At the same time, use of the invention saves time and money for the people working the land with no need to directly mix or handle diesel or other hydrocarbon carrier, or the potentially toxic active ingredients.

The following describes non-limiting examples of the disclosure being used with reference to a treatment block, apparatus and method for treatment of cattle on farmland, against biting insects, as a particularly useful application of the invention. However, the treatment block, apparatus and method may be adapted for use for application of any treatment to any animal. It is not intended to limit the invention in any way, other than as stated in the claims, to cattle.

Throughout the specification and claims the term "treatment" is used as a useful general term for the application of any suitable active ingredient or additive to an animal against the action of biting insects, as would be readily understood by a person skilled in the art. It is not intended that the word "treatment" be in any way limiting, other than as defined in the claims.

The term carrier medium is used to refer to the single or blended materials used to carry the active ingredient. As described this is typically a blend of wax and oils to create a block of suitable characteristics. The carrier medium may be any suitable carrier medium that includes synthetic high temperature hard wax and it is not intended to limit the carrier medium to the wax and other blends described, other than as specifically defined in the claims.

Wax is a very useful product for the invention, and the block may be made of a mix of waxes, or materials that act like wax, or such materials with other materials, whether they be natural or synthetic. Other materials may be used and it is not intended to limit the invention to wax, other than as stated in the claims. However, use of wax is most advantageous for the invention, and in particular the wax mixes determined by the inventor as the best means to be perform the invention at the present time.

For clarity, any prior art referred to herein, does not constitute an admission that the prior art forms part of the common general knowledge, in Australia or elsewhere.

It is an object of the present disclosure to provide an apparatus for treatment of animals that at least ameliorates one or more of the aforementioned problems of the prior art. It is an object of the present invention to provide a treatment block that at least ameliorates one or more of the aforementioned problems of the prior art. It is another further, separate, object of the present disclosure to provide a method of treatment of animals that at least ameliorates one or more of the aforementioned problems of the prior art.

### DISCLOSURE OF THE INVENTION

Accordingly, the present invention provides an animal treatment block according to claim 1 for applying a treatment to an animal against the action of biting insects, the animal treatment block including:
a body including a carrier medium; and is characterised by the features set out in the characterising portion of the main claim.

The animal may be any animal. Preferably, the animal is livestock. Most preferably, the animal is cattle. The cattle may be any age, type or breed of cattle kept for any purpose. The cattle may be chosen from the group: beef cattle; breeders; calves; and dairy cattle. The animal may be a horse or pony. The animal may be deer or similar. The animal may be a goat. Preferably, the animals are farmed. The animals may be hobby farm or pet animals in other forms of the invention.

The biting insects may be any insects that bother or annoy an animal, not limited to those that bite or draw blood. The biting insects may be chosen from the group: buffalo flies; horn flies; sucking lice; biting lice; face flies; and horse flies. The treatments may act immediately to kill insects on contact. The treatments may act to disrupt the life cycle of the insects. The insects may be killed in any suitable manner. The insects may be deterred from approaching a treated animal in some forms of the invention, for example where an essential oil is included.

The animal treatment block of the invention is a substantially cylindrical block suitable to be used as a roll or rub to apply the treatment to the animal. A cylinder block is a useful configuration for manufacture, sale, transportation and generally handling. Most preferably, the animal treatment block is configured to be a convenient size to handle by the user. Most preferably, the animal treatment block is adapted to be convenient and safe to handle, the active ingredients being maintained in a substantially solid form. The transfer or release of the active ingredient may be controlled by the carrier medium being in substantially solid form. The rolling or rubbing of the cylinder block against the skin of an animal applies the treatment in a controlled manner. The control can be through the rolling and through the slow transfer of the material from the block to the animal. The control contrasts to the uncontrolled liquid soaked application methods of the prior art. Preferably, a plurality of animal treatment blocks will be supplied to the end user, for use together. A stock of animal treatment blocks may be provided. Typically 6 to 10 animal treatment blocks may be used together. Packs of 6 to 10 animal treatment blocks may be provided to the end user. Or individual units may be made available for sale. It is advantageous that the length of treatment area may be increased or decreased through use of more or less of the treatment blocks to suit the needs of the user. A plurality of blocks may be used to provide an extended treatment area to contact the animal, to increase the surface area of possible contact area with the animal.

Preferably, the body is substantially a cylindrical shape. The body may be any suitable shape. A generally cylindrical shaped body is beneficial as it enables the animal to roll the body of the animal treatment block onto its body. The rub is in this way reminiscent of trees, branches or posts on which an animal may naturally scratch or rub. Most preferably, the body of the animal treatment block is adapted to be a shape that can be mounted and once mounted an animal can rub itself on the body. The animal may also rub against the body in any manner and active ingredient is applied to the animal in the carrier medium.

The contact and or rolling action assists to apply a layer of the carrier medium with active ingredient to the animal. Preferably, the rate of delivery is at least to some extent controlled by the structural means. Preferably, the rate of application is controlled by the structural means, slowing the breakdown of the block.

The body includes a carrier medium for the active ingredient. The carrier medium is preferably, the main constituents making up the block excepting the active ingredient and structural means. In a preferred form the active ingredient and structure means are mixed in the carrier medium to create the block. In other forms of the invention the active ingredient may be mixed or incorporated with other materials before being mixed with the carrier medium. For example, the active ingredient may be mixed with the structural means before being mixed with the carrier medium. Any carrier medium that includes a synthetic high temperature hard wax can be mixed with the structural means and an active ingredient in any manner.

The body includes a substantially solid carrier medium. The substantially solid carrier medium is of a suitable hardness or softness to transfer material to an animal when rubbed or contacted. The carrier medium is not so soft or liquid so as to drip on the ground or be released in similar uncontrolled manners. Preferably, the carrier medium can be in liquid form, or a softened form during manufacture for incorporation of the active ingredient and structural means, where used. The carrier medium may be softened during manufacture before mixing with the other components. The body is formed of carrier medium and other components that together form the body of the block. The body includes a carrier medium and other materials mixed together, including the active ingredient.

The body is configured whereby in use some of the carrier medium and carried active ingredient from the body will be transferred to the animal when the animal rubs or otherwise contacts the body, in a controlled manner. As the animal contacts the body, part of carrier medium is applied to the animal, applying the active ingredient to the animal, treating the animal against the biting insects. The body includes a carrier medium substantially made of a material that is substantially solid at room temperature but can be readily returned to a liquid form through use of heat. The carrier medium of the animal treatment is substantially solid in use but loses some material to the animal as the animal rubs or contacts the carrier medium whereby some of the carrier medium and incorporated active ingredient are applied to the animal. The body includes a carrier medium for the active ingredient that is useful to be maintained in a controlled manner until rubbed by the animal, whereby a suitable amount of carrier medium and incorporated active ingredient are applied to the animal. The amount of carrier medium transferred and the volume of contained active ingredient may be varied to suit the particular needs of the animal.

The control is the application only on contact with the animal rather than uncontrolled dripping as found in liquid carriers. In this way the insecticide can be applied to the animal from the body of the animal treatment means to the animal to act against the action of biting flies as it is needed, in a controlled manner.

Any suitable material that includes a synthetic high temperature hard wax may be used for the carrier medium. The carrier is substantially solid at room temperature. The carrier medium is readily returned to a liquid or semi-liquid state through use of heat. Preferably, the carrier medium is solid at below 30 degrees Centigrade. Preferably, the carrier medium is solid at 32 degrees Centigrade. Preferably, the carrier medium is solid at 35 degrees Centigrade. Preferably, the carrier is generally an inert material, safe for the environment. Preferably, the carrier may be softened to be mixed with other materials, and or the active ingredient and set into a block for use. Most preferably, the carrier medium is readily softened through use of heat, and on returning to room temperature sets to a solid block. Preferably, the carrier medium is a solid material in use that readily loses some material on to the animal, including the incorporated active ingredient when rubbed or contacted. The carrier medium may be adapted to soften to an oily paste or similar, in use, whereby a small amount will be applied to the animal on contact, so as to treat with the active ingredient. However, during manufacture, the carrier medium may also be soften suitably to enable ready incorporation of the structural means and active ingredient.

Preferably, the carrier medium is substantially a wax block. Preferably the carrier medium includes paraffin oil. Preferably, the carrier medium includes substantially 50 percent paraffin oil. Preferably, the wax is mixed with the active ingredient before the block is set, ready for use. Preferably, the mixing enables even distribution of the active ingredient throughout the body.

According to the disclosure, the wax may be chosen from the group: bee wax; natural wax; palm wax; paraffin wax; microcrystalline wax; soy wax; SASALWAX H1 (Trade mark) and synthetic wax. However the invention as defined in claim 1 requires the use of a synthetic high temperature hard wax.

In one form of the invention paraffin wax is used, with a melting point of substantially 62 degrees Centigrade. The paraffin wax may be replaced with another material with similar properties and melting temperature. Preferably, the paraffin wax is included in to increase the oil content which assists in the transfer to the animal. In one form of the disclosure palm wax is used with a melting point of substantially 72 degrees Centigrade. The palm wax may be replaced with another material with similar properties and melting temperature. Where palm wax is included, this may be to increase the hardness of the block. Preferably, the synthetic high temperature hard wax has a melting temperature of substantially 105 degrees Centigrade.

The synthetic high temperature hard wax is included to raise the melting temperature of the carrier medium to resist melting in hot conditions. Most preferably, the wax is a mix or blend of waxes. Preferably, the blend or mix of waxes are chosen for their combined specific properties. Most preferably, the wax is a blend of suitable waxes chosen for the particular application and characteristics. The characteristics may include melting point; transfer of material; brittleness; and easy of mixing during manufacture. Cost may be a factor for consideration, as synthetic waxes and microcrystalline waxes are useful to include, but are of a higher cost than some other waxes. Ideally a blend of wax is used with give a balance of functionality and cost of production. Preferably, different forms and versions of the block will be made available with different wax blends as appropriate. Particularly useful block blends will be provided for the most useful situations. The weather and ambient temperature of the location of use will be an important consideration. Preferably, a wax blend will be provided suitable to use up to 35 degrees Centigrade. The wax blend in this form will be useful in most situations. However, it is envisaged that another wax blend may be provided, suitable in particular for use at 35 degrees Centigrade and above. A higher proportion of synthetic high temperature wax may be used in this case, to increase the melting point. SASOLWAX H1 (Trade Mark) may be used. SASOLWAX H1 (Trade Mark) may be used in combination with microcrystalline wax. The wax mixtures may be as defined in Table 1, for any of the described blocks or similarly as defined in Table 2 in any of the blocks.

Most preferably, different wax blends are used with different active ingredients to provide suitable products for different applications.

Preferably, a wax blend is used. Most preferably, a wax blend including white oil, petroleum jelly, and synthetic wax is used. Preferably, the wax blend includes white oil and or petroleum jelly in addition to the wax. Preferably, a blend of paraffin oil, petroleum jelly, with a microcrystalline wax is used. Preferably, a synthetic wax just as Sasolwax H1 is also included in the blend. In a variant, a wax blend of paraffin wax, microcrystalline and synthetic wax may be used. Preferably, in a further variant, the wax blend is of paraffin wax and synthetic wax. Preferably, in another further variant, the wax blend is of palm wax and synthetic wax. Preferably, a wax blend is chosen to give suitable properties of the wax carrier to provide a block with an appropriate melting point, and hardness that is resistant to breakage, and enables a controlled transfer of the active ingredient as the animal rubs against the block.

According to every form of the invention synthetic high temperature hard wax is included in the wax blend. The synthetic high temperature hard wax may increase the melting point. Preferably, the synthetic high temperature hard wax replaces part of the palm or paraffin wax in the blend. Synthetic high temperature hard wax may be used in the range of 5 to 50%. The synthetic high temperature hard wax may be a product such as FISCHER-TROPSCH (Trade Mark) or SHELL (Trade Mark). The synthetic high temperature wax may be SASOLWAX H1 (Trade Mark). Any suitable synthetic high temperature hard wax may be included in the carrier medium. The wax may be adapted for particular heat conditions. For example, a wax blend may be used that is suitable for use up to 30, 32 or 35 degrees Centigrade. Other forms of the invention may be adapted so that the wax blend has a melting temperature suitable for use at above 35 degrees Centigrade. Other blends may be resistant to melting at greater temperatures. For cold environments, different wax mixtures may be used to soften the material to encourage transfer of the carrier medium and associated active ingredient onto the animal. White oil, paraffin oil is preferably mixed with the wax to adapt the consistency, for manufacture or to assist transfer.

Preferably, in one form of the invention the percentages are as follows 32.2 percent synthetic high temperature wax, 5.4 percent microcrystalline wax, 6.2 percent petroleum jelly, 50.8 percent mineral oil (light paraffin oil also known as white oil) with 2.9 percent coconut coir and a 2.5 percent of active ingredient such as chosen from diazinon, chlorfenvinphos or phosmet.

Preferably, in another form of the invention the percentages are as follows 32.2 percent synthetic high temperature wax, 5.4 percent microcrystalline wax, 6.2 percent petroleum jelly, 51.4 percent mineral oil (light paraffin oil also known as white oil) with 2.9 percent coconut coir and a 1.9 percent of active ingredient permethrin.

Preferably, for a further other form of the invention the percentages are as follows 32.2 percent synthetic high temperature wax, 5.42 percent microcrystalline wax, 6.27 percent petroleum jelly, 50.62 percent mineral oil (light paraffin oil also known as white oil) with 2.97 percent coconut coir and a 2.52 percent of active ingredient as follows, eucalyptus oil 1.44 percent, tea tree oil 0.21 percent, lemon grass oil 0.08 percent times 2, lavender oil 0.08 percent, aster amellus root juice 0.04 percent, pine oil 0.04 percent, citronella oil 0.21 percent, palmarosa oil, 0.08 percent and clionasteral 0.34 percent.

Preferably, for another form of the invention, the percentages are as follows 32.29 percent synthetic high temperature wax, 5.42 percent microcrystalline wax, 6.27 percent petroleum jelly, 50.51 percent mineral oil (light paraffin oil also known as white oil) with 2.97 percent coconut coir, 1.44 percent eucalyptus oil, 0.21 percent tea tree oil, 0.08 percent lemon grass oil, and 0.81 percent Citronella oil.

Most preferably, substantially 32.2 percent of a high temperature synthetic wax is included in the carrier medium. Most preferably, substantially 5.4 percent of microcrystalline wax is included in the carrier medium. Most preferably, substantially 32.2 percent of a high temperature synthetic wax and substantially 5.4 percent of microcrystalline wax is included in the carrier medium. Preferably, substantially 6.2 percent petroleum jelly is used in the carrier medium. Preferably, substantially 51 percent paraffin oil is used in the carrier medium. Most preferably, substantially 32.2 percent of a high temperature synthetic wax and substantially 5.4 percent of microcrystalline wax is included in the carrier medium, with substantially 6.2 percent petroleum jelly and substantially 51 percent paraffin oil is used in the carrier medium.

Most preferably, wax is used as a carrier medium to which active ingredient is added, with structural means. The wax is preferably a blend of microcrystalline wax and Sasolwax H1 (Trade Mark). This preferred mixture also preferably is mixed with paraffin oil and petroleum jelly to achieve an appropriate hardness for use.

Preferably, the body is formed from a mixture of wax and active ingredient that is mixed in a softened state, and formed into a suitable shaped block. Preferably, one or more mould is used to shape the body. The mould may be any suitable shape. Preferably, the mould or moulds enable a plurality of blocks to be made at the same time. Most preferably, the moulds produce a cylindrical body. Preferably, the mould include shaping for a conduit through the body. Most preferably, the mould is cylindrical with means to create a cylindrical conduit through the body.

Preferably, the body includes shaping to assist in mounting of the body in use. Preferably, the body includes a conduit to enable the body to be strung, hung or mounted. Preferably, the body includes a conduit through the body to enable the body to be hung or otherwise suspended for use. Preferably, the body includes a substantially cylindrical conduit. Preferably, the body is substantially cylindrical and the conduit is also substantially cylindrical, which is beneficial to hang the body for use. The conduit may take any suitable form. Other means to mount the body may be used instead. In an inferior form of the invention, attachments may be made to each side of the block to mount them instead.

Preferably, the block includes means to resist breakdown due to exposure to the sun. A UV inhibitor may be included in the body. A UV inhibitor may be included in the carrier medium. The UV inhibitor may be 2-Hydroxy-4-n-octoxybenzophenone. Any suitable Benzophenone UV inhibitor may be used. Other suitable UV inhibitors to act against degradation through sun exposure may be used instead The UV inhibitor may act to resist against the action of the sun in use. Preferably, the UV inhibitor is mixed through the carrier medium during manufacture. Substantially 0.005% of UV inhibitor may be used per block. Any suitable volume or concentration of UV inhibitor may be used instead, to act against degradation due to UV.

Most preferably, the body is a block of wax carrier medium with the active ingredient incorporated in when the wax is in a melted liquid state, set in a cylindrical shaped mould, adapted to form a conduit through it, to produce a suitable shape for use to be rolled and contacted on by an animal, when mounted. The wax carrier medium may be wax or a mixture of wax and other ingredients.

The active ingredient may be any suitable active ingredient to act against insects. Preferably, the active ingredient is an insecticide. In other forms of the invention the active ingredient may be any useful additive it is desired to apply to the animal. In this form of the invention the additive may be to improve the health or wellbeing of the animal in any way, not limited to acting against insects.

The active ingredient may be or include any suitable synthetic pyrethroid. The active ingredient may be or include any suitable organophosphate. The active ingredient may be or include any suitable essential oil.

Preferably, a single active ingredient is included. A plurality of active ingredients may be used, in other forms of the invention. A blend of active ingredients may be used. Where the active ingredient includes essential oils a blend of suitable essential oils may be used. The active ingredient may be synthetic. The active ingredient may be a naturally occurring product. The active ingredient may be chosen from the group: permethrin; chlorfenvinphos; coumaphos; diazinon; phosmet citronella; eucalyptus; or other essential oils. Any suitable essential oils, or combinations of essential oils may be used. Any suitable combination of essential oils may be used to discourage insects. The essential oils or similar products may include any one or more chosen from the following group: eucalyptus; tea tree; lemon grass; lavender; aster amellus root juice; pine; citronella; palmarosa; clionasteral; or similar. The active ingredient may be provided through adding a blend of ingredients, which themselves include one or more suitable active ingredients. For example, products such as BRUTE ("Trade Mark") which contains permethrin, SUPONA ("Trade Mark") which contains chlorfenvinphos, Diazinon (product name and active ingredient), or IMIDAN ("Trade Mark") which contains phosmet, may be incorporated to introduce the active ingredients into the carrier medium. These are example branded products and the active ingredients may be provided in any suitable form, not limited to those branded formulas listed.

The active ingredient may be a proprietary blend useful for treatment of animals. The proprietary blend may include one or more active ingredients. The proprietary blend may be chosen from the group: HORSE CARE ("Trade Mark"); and CATTLE CARE ("Trade Mark"). Different blocks with different mixes of wax or different active ingredients may be used together in some forms of the invention. Generally however several blocks with the same active ingredient will be used as suitable for the particular animal and particular conditions.

Preferably, the volume of active ingredient incorporated is suitable to treat the animal. For example, where the block is 1700 millilitres, the volume of active ingredient may be 150 millilitres of SUPONA ("Trade Mark") containing the active ingredient Chlorfenvinphos at a concentration of 200 grams per litre. Preferably, where the block is 1180 millilitres, the volume of active ingredient is 30 millilitres of the active ingredient Chlorfenvinphos at a concentration of 200 grams per litre where the block has a volume of 1180 millilitres. Any suitable volume and concentration may be used.

Preferably, where the block is 1700 millilitres, the volume of active ingredient may be 150 millilitres of the product sold as Diazinon and containing the active ingredient Diazinon at a concentration of 200 grams per litre Preferably, where the block is 1180 millilitres, the volume of active ingredient is 30 millilitres of the active ingredient diazinon at a concentration of 200 grams per litre where the block has a volume of 1180 millilitres. Any suitable volume and concentration may be used.

Preferably, where the block is 1700 millilitres, the volume of active ingredient may be 20 millilitres of BRUTE ("Trade Mark") containing the active ingredient Permethrin at a concentration of 87 grams per litre. Preferably, where the block is 1180 millilitres, the volume of active ingredient is 22 millilitres of the active ingredient Permethrin at a concentration of 87 grams per litre where the block has a volume of 1180 millilitres. Any suitable volume and concentration may be used.

Preferably, where the block is 1700 millilitres, the volume of active ingredient may be 20 millilitres of IMIDAN ("Trade Mark") containing the active ingredient Phosmet at a concentration of 150 grams per litre. Preferably, where the block is 1180 millilitres, the volume of active ingredient is 30 millilitres of the active ingredient Phosmet at a concentration of 150 grams per litre where the block has a volume of 1180 millilitres. Any suitable volume and concentration may be used.

Preferably, where the block is 1700 millilitres, the volume of active ingredients are derived from the HORSE CARE ("Trade Mark") blend is 1000 millilitres. Any suitable volume and concentration may be used. Preferably, where the block is 1700 millilitres, the volume of active ingredient may be 63 millilitres of citronella oil and 37 millilitres of eucalyptus oil containing the active ingredient concentrate. Other formulations may be used including other essential oils in combination. Preferably here the block is 1180 millilitres, the volume of active ingredient is 2.5 millilitres of citronella oil and 17 millilitres of eucalyptus oil with one or more of 2.5 millilitres of tea tree oil, 1 millilitres of lemon grass oil, 1 or 2 millimetres of lavender oil, 0.5 millilitres of aster amellus root juice, 0.5 millilitres of pine oil, 1 millilitres of palmarosa oil, and 4 millilitres of clionasterol. Most preferably, all of the aforementioned are included with 1 millilitres of lavender oil. Any suitable volume and concentration may be used.

Preferably, where the block is 1700 millilitres, the volume of active ingredients are derived from the CATTLE CARE ("Trade Mark") blend is 1000 millilitres. Any suitable volume and concentration may be used or where the block is 1180 millilitres, the volume of active ingredients may be eucalyptus oil 17 millilitres, tea tree oil 2.5 millimetres, lemon grass oil or lavender oil 1 millilitres and citronella 9.5 millilitres per 1180 millilitres block.

Any suitable other ingredients may be included in the mix to make the body. According to the invention the body includes structural means including a plurality of fibres. The structural means may take any suitable form. Preferably, the structural means makes the block more resilient in some way. Or the structural means may lengthen the life of the block. For example, the structural means may assist to maintain the shape and structure, such as during manufacture or use, while the carrier medium readily transfers to the animal to apply the treatment to the animal. The structural means may substantially protect the block during transportation and storage. The structural means include a plurality of fibres and may be inclusion of any suitable material that gives the block stronger resilience or strength in some way, compared to a material without the structural means.

The structural means include a plurality of fibres which are mixed into the carrier medium of the body to give structural integrity. The fibres may be any suitable fibres. The fibres may be coconut coir. Coconut coir is a useful strong, light material to add structure to the block. The fibres may be replaced with any suitable particulates or fibrous material. The fibres may be long fibres or short fibres. In a preferred form of the invention, the fibres are coconut coir fibres, as found suitable for the application. The fibres may be processed to be short, long or a mix of sizes of fibres to suit the application. Particulates and fibres may be used as structural means in some forms of the invention.

Preferably, the coconut coir fibres are substantially 150 to 200 millimetres in length. For example, substantially 20 grams or a volume of 66 millilitres of coconut coir are mixed in per 1700 millilitre block in a preferred form of the invention. Where the block is 1180 millimetres block 35 millilitres of coconut coir per block may be used. The volume of coconut coir equates to 12 grams in the 1180 millilitre block. Although some variation may be made the particular percentages have been carefully determined through research to appropriately assist in the treatment application at a controlled rate. Coconut coir may be mixed in at a percentage of substantially 2.9%. Coconut coir may be mixed in at a percentage of substantially 4%, in other forms of the invention. A greater or lesser percentage of coconut coir may be used instead. Preferably, the structural means provides structural stability to the block in use. The structural means may be adapted to suit the particular application of the block in use.

Preferably, the incorporation is the mixing of the structural means into the carrier medium during manufacture. The active ingredient and structural means may both be incorporated into the carrier medium in a softened or liquid state before the block is set into a substantially solid block.

According to the disclosure, the body may be formed according to the mixtures of Table 1 or 2 as for any of the illustrated example blocks. Other materials may also be incorporated into the body for structural purposes or to adapt the construction properties.

The block is configured to enable use for several weeks, to provide treatment for animals for several weeks. The block may be adapted to be useful to provide treatment for animals for more than 12 weeks. The invention may enable the apparatus to be installed to provide more than 12 weeks of treatment against insects of the animals without further input. The block may be adapted to be useful to provide treatment for animals for more than 20 weeks. The invention may enable the apparatus to be installed to provide more than 20 weeks of treatment against insects of the animals without further input. There is a significant advantage in the use of the invention to act against biting flies and other biting insects without the need for regular attendance by the cattle producer, whereby the treatment for the cattle to improve their health, comfort and potential sale price continues at all times.

The block may be mounted using any suitable means. Preferably, the mounting the positioning of the block at a suitable height and location for convenient contact by the animal to transfer the carrier medium and incorporated active ingredient to the animal. Preferably, the block includes a conduit and a longitudinal material is used through the conduit to hang the block. Preferably, a plurality of blocks are hung in this way. Preferably, a plurality of blocks are used together. Preferably, the number of blocks used varies the length of contact area for an animal. A single block may be used in some forms of the invention. Preferably, 6 to 10 blocks are strung together on a rope or the like between two points. The points may be posts or poles. The points may be trees. Preferably, the block includes a conduit and a plurality of blocks are strung together on a rope or chain through the conduit between two points.

Preferably, the mounting of the blocks occurs at a location where the animals congregate. Preferably, the animals are cattle and blocks are mounted at locations where the cattle congregate which may be near water or shade. Preferably, the blocks are hung at a height on which the animals can comfortably rub or scratch themselves. Preferably, the blocks are hung at a level substantially below the typical height of the back of the animal. Most preferably, the animal can readily contact their back, neck or head on the mounted blocks.

When the block or blocks are mounted these may be contacted by the animal and part of the body of the block with incorporated active ingredient is applied to the animal. The carrier with the incorporated active ingredient is applied to the animal. The wax is readily applied to the animal as it rubs itself on the body and so the active ingredient is applied to the animal. Preferably, the carrier medium is a wax blend suitable to apply the active ingredient to the animals in a controlled manner. Preferably, the blocks are mounted where the animals rub, and scratch themselves to rid themselves of biting insects whereby they contact the carrier medium and the active ingredient is transferred and applied. Preferably, the blocks are mounted where the animals rub, and scratch themselves to rid themselves of biting insects whereby they contact the carrier medium and the active ingredient is transferred and applied, and the cylindrical shape of the blocks rolls the carrier medium and active ingredient for controlled application as the animal rubs or scratches.

The contact may be any suitable contact. The animals use the mounted apparatus to rub, and scratch themselves to rid themselves of biting insects whereby they contact the carrier medium and the active ingredient is transferred and applied. As they do so active ingredient is applied by means of the carrier to give them relief from the insects for a few days. As the animals revisit the apparatus, when again they need to rub due to insects, a further dose or application is received to again apply the insecticide and provide relief. Prior art apparatus uses a liquid carrier for insecticide which can drip and is uncontrolled in its use. The control of the invention may be through use of a substantially solid carrier medium. The control may be transfer of carrier medium and incorporated active ingredient only on contact by the animal. The control may be varied due to the properties of the carrier medium. For example, where the invention is to be used at high temperatures the high melt point of the carrier medium controls so an appropriate amount of carrier is transferred. The block in this form may be too hard for use in cold environments as perhaps too little carrier medium would be transferred. For cold environments a carrier medium with a lower melt point will be used instead to enable a controlled transfer of an appropriate amount of carrier medium and incorporated active ingredient.

The control may be to enable an appropriate amount of carrier medium with incorporated active ingredient to be transferred. The control may be to enable an appropriate amount of carrier medium with incorporated active ingredient to be transferred for the environmental conditions. The block is a solid block, whereby there is little or no uncontrolled loss of active ingredient. Preferably, the solid block maintains the active ingredient in the block, substantially preventing the accidental loss of material and in particular active ingredient other than when rubbed by an animal. The body includes a wax carrier and the wax carrier maintains the active ingredient in a controlled manner, so that it cannot be accidentally inhaled or imbibed. Instead the wax carrier and active ingredient may be maintained in the body of the block until rubbed by the animal, whereby application occurs. Most preferably, the controlled manner is substantially the prevention of accidental loss of carrier medium. Most preferably, the controlled manner is the determination of the amount of carrier medium and hence active ingredient transferred on contact. The amount can be increased or decreased through use of a carrier medium with different properties, such as the melt point and hardness in a solid state.

The body is adapted to give an extended useful life to the block. The block is able to provide relief against biting insects for more than 2 weeks. The block may be able to be functional for more than a month. The block may be functional for one, two, three or more months. The block may provide useful treatment for more than12 weeks. Preferably, the block provides useful treatment for up to 20 weeks. Preferably, the block provides useful treatment for more than 20 weeks. Shorter or longer term blocks may be provided in some forms of the invention.

Preferably, the volume of the blocks is substantially 1180 millilitres, and 100 millimetres wide by 145 millilitres long. The particular dimensions of the block can be varied, but these dimensions have been found suitable for use.

Preferably, the block is made according to the volume data detailed below in one or more part of Table 1 or Table 2, as identified for a particular active ingredient or ingredients.

Preferably, a bush is included through the block. The bush may form part of the structural means. Preferably, the bush protects the block from undue damage when mounted. Preferably, the bush is associated with a core of the block and protects the core from undue damage when mounted. Where mounted on a chain or similar, the bush protects the core and or ends of the block from wear during use. The protective bush may be made of any suitable material. Preferably, the material is biodegradable so that when the block has been used the spent bush or core does not negatively impact the environment. Any suitable material may be used the material may be chosen from the group: bamboo; natural plastic; wood; or any other suitable biodegradable material.

In another, non claimed form of the disclosure there may be an animal treatment block for applying a treatment to an animal against the action of biting insects, the animal treatment block including:
a body including a wax carrier;
an active ingredient mixed with the wax carrier; and
a fibrous structural means incorporated into the wax carrier,
wherein the body may be held in the hand of the person and applied to the animal to apply the active ingredient in the wax carrier to the animal in a controlled manner.

Accordingly, the present disclosure also provides a non claimed apparatus for applying a treatment to cattle against the action of biting insects, the apparatus including:
a body including a wax carrier;
a mounting means to mount the body above the ground so as to be at a suitable position whereby cattle can contact the body;
an active ingredient incorporated into the body to act as an insecticide against the biting insects; and
a structural means incorporated or part of the body being coconut coir,
wherein the mounting of the body is such that cattle can contact the body by rubbing so as to apply the carrier and incorporated insecticide, and the application is in a controlled manner due to the body being in a substantially solid form.

Accordingly, the disclosure also provides a non claimed method of using an animal treatment block for applying a treatment to an animal against the action of biting insects, the treatment block including a body, and an active ingredient incorporated into the body, the method including the following steps:-
a) mounting the block at a suitable location;
b) rubbing of the animal on the body of the block, typically on the back, head or neck;
c) transferring part of the body of the block with incorporated active ingredient to the animal,
whereby the active ingredient acts against the action of biting insects and the transfer is in a controlled manner.

Accordingly, the disclosure also provides a non claimed method of using an animal treatment block for applying a treatment by hand to an animal against the action of biting insects, the treatment block including a body, and an active ingredient incorporated into the body, the method including the following steps:-
a) applying the body of the animal treatment block to the animal; and
b) contacting the body such that part of the body with incorporated active ingredient is applied to the animal;
whereby the active ingredient acts against the action of biting insects in a controlled manner.

The block of the apparatus may be the block of any of the forms or variants of the disclosure. The block of the methods may be the block of any of the forms or variants of the disclosure.

### INDUSTRIAL APPLICABILITY

The animal treatment apparatus blocks can be manufactured including industrially, and provided to wholesalers or retailers for on-sale, or to customers directly for use.

### BRIEF DESCRIPTION OF THE DRAWINGS

The invention will now be described in connection with non-limiting preferred embodiments with reference to the accompanying drawings, in which:
Figure 1 is a perspective view from above of a cylinder block according to a first preferred embodiment of the invention for use on a hanging apparatus (not shown other than in Figure 7, for ease of illustration);
Figure 2 is an end view of the cylinder block of Figure 1;
Figure 3 is a side view of the cylinder block of Figures 1 and 2;
Figure 4 is a perspective view from above of a cylinder block according to a second preferred embodiment of the invention for use on a hanging apparatus (not shown for ease of illustration);
Figure 5 is an end view of the cylinder block of Figure 4;
Figure 6 is a side view of the cylinder block of Figures 4 and 5;
Figure 7 is a perspective view from above of a cylinder block according to a third preferred embodiment of the invention for use on a hanging apparatus (not shown for ease of illustration) with biodegradable bush;
Figure 8 is an end view of the cylinder block of Figure 7;
Figure 9 is a side view of the cylinder block of Figures 7 and 8;
Figure 10 is a further perspective view from above of the cylinder block with biodegradable bush of Figures 7 to 9, showing the internal structure;
Figure 11 is an end view of the cylinder block of Figure 10;
Figure 12 is a side view of the cylinder block of Figures 9 and 10; and
Figure 13 is a schematic diagram illustrating the cylinder blocks of any of Figures 1 to 12, and others in use on a hanging apparatus.

### DETAILED DESCRIPTION OF THE INVENTION INCLUDING A BEST MODE

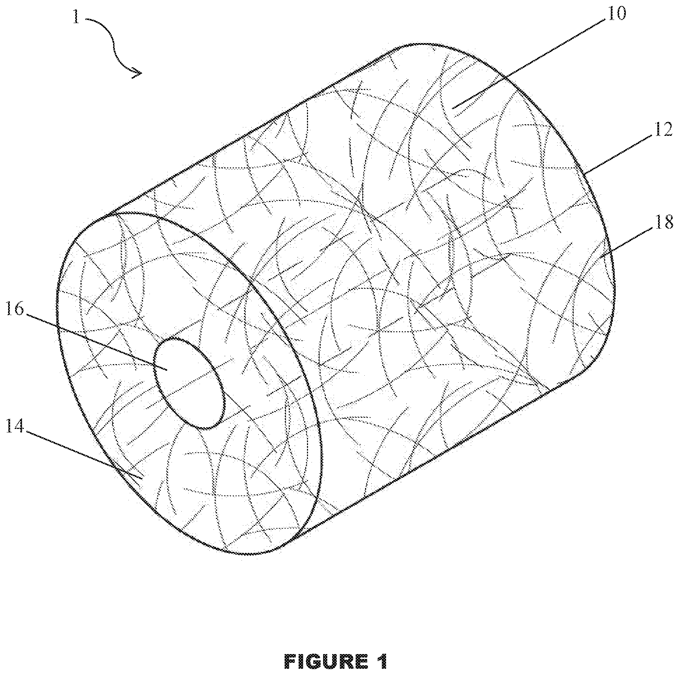

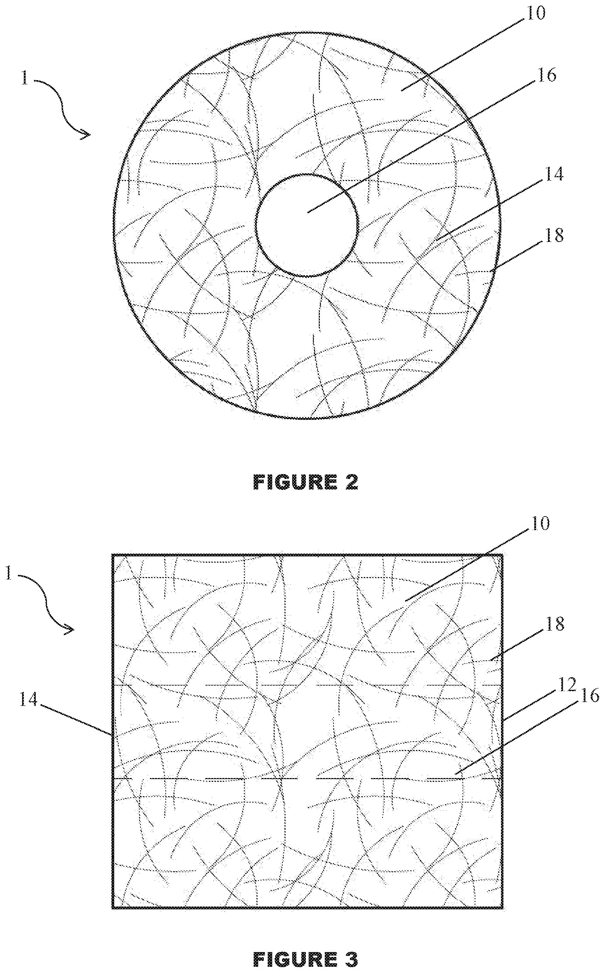

Referring to Figures 1 to 4 and 13, a first preferred embodiment of the invention will be described, where cylinder block 1 has body 10, with ends 12 and 14. As illustrated each body 1 is 134 millimetres wide by 121 millimetres wide. The size is convenient to hold in the hand of the person so may be readily manipulated at the time of purchase, transit, storage or use. In use, several cylinder blocks 1 are used together to create a length of treatment area suitable for the particular animals, for example 6 to 10 of cylinder blocks 10 may be used for cattle. The use of 6 to 10 cylinder blocks creates an extended area of contact for the animal to enable transfer of material. Where the animal is smaller less cylinder blocks may be used instead. It is particularly useful for adaptability for the body to be short and squat such that any suitable length of treatment area can be created, and with reduced risk of damage in transit. It is envisaged, however, that longer lengths could be manufactured instead and supplied ready for use, within the scope of the invention. In another form of the invention, not described here, the blocks are used to manually apply treatment to the animal. These smaller blocks would be configured to fit conveniently in the hand and applied by contact with the animal. Cylindrical conduit 16 (described below) may be omitted in this alternative form of the invention. In this way the novel treatment block may be used with a wide range of treatments for a wide range of animals.

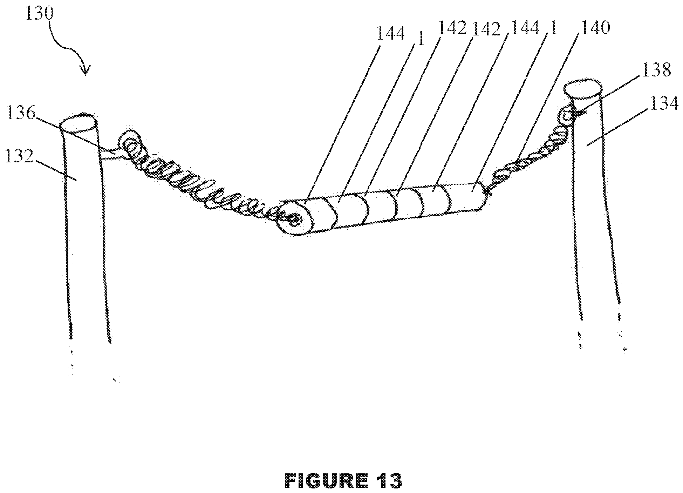

Cylindrical conduit 16 runs through the centre of body 10 of cylinder block 1 from end 12 to end 14. It is by means of cylindrical conduit 16 that several cylinder blocks 1 are hung for use, as described further below and with respect to Figure 13. Cylindrical conduit 16 is 32 millimetres in diameter as illustrated.

Cylinder block 1 is manufactured from an inventive mix of wax, reinforcing and active ingredients. A different mix each of wax, reinforcing and active ingredients may be used for different applications, that is different animals, insect needs or environmental conditions, but the principle of the invention is the same throughout. The different blends of carrier, structural materials and active ingredients are in particular set out in Table 1 and Table 2 below.

Block 1 is illustrated made manually, poured into a suitable container of a suitable shape, and set. Once set the block can be removed and retains the shape of the container. The manual process can be quickly repeated for repeated blocks. It is also anticipated that the mechanism will be automated, through the manufacturing process to produce multiple blocks quickly and easily.

For Cylinder block 1 the first form of the invention, as described, the wax is a mix of synthetic high temperature hard wax, paraffin wax and palm wax in a ratio of 35% synthetic high temperature hard wax, 30% paraffin wax and 35% palm wax. The described illustrative ratio is useful for applications up to 35 degrees Centigrade. Each type of wax is chosen and balanced for the wax attributes. For example, in the above mix, the synthetic high temperature hard wax is included to increase the melting temperature of block 1, having a melting point of 105 degrees Centigrade, a very high melting point for a wax material. The paraffin wax is included for the added oil content, to aid transfer of the active ingredient to the animal. The paraffin wax and palm wax both have a lower melting point at around 62 degrees Centigrade. The palm wax increases the overall hardness of the block, but would make the block too brittle if used in a high proportion.

In other forms of the invention where the block is to be used at temperatures over 35 degrees Centigrade an alternative mixture may be used with 50% synthetic high temperature wax, 25% palm wax and 25% paraffin wax. In other blends, microcrystalline wax may be included, which has a melting point around 72 degrees Centigrade, higher than the paraffin or palm wax. However, use of the synthetic high temperature wax is preferred, if the cost is appropriate. Clearly, the blend mix can be varied somewhat for different applications. Further wax blends and proportions are used in other forms of the invention as described below.

The described example mix is useful to at least 35 degrees Centigrade. Other blends and ratio mixes are also being investigated for their relative properties for use as an inventive block, in higher heat conditions. For example, for conditions over 35 degrees Centigrade incorporation of a greater proportion of synthetic high temperature wax product such as FISCHER_TROPSCH (Trade Mark) into the mixture can be used as it has a very high melting point. The inventor has found that specific combinations of waxes with their different melting points and properties is extremely beneficial to create cylinder block 1 with the best application of the active ingredient and longevity in the field.

Fibre 18 is incorporated into the mixture while the waxes are softened into a liquid form so that the fibres become thoroughly combined. The fibre 18 as illustrated is coconut coir separated into fibres and mixed through. This is an environmentally sound product that is lightweight but strong, giving a strength and structure to the wax. Coconut coir of 20 grams per block is used, or 66 millilitres (refer also to the TABLE 1 mix examples below) to give a suitable reinforcement and structure to the wax block, and Table 2 gives further possible blend examples. Some variation to this could also be used. It is envisaged that any suitable fibre could be used instead, but it has been found that coconut coir is particularly useful.

The structure assists to hold the wax block together, resists release of the active materials. It is an important advantage of the subject invention that the use is controlled as the animal contacts the block. The controlled use compares to the uncontrolled use of a hydrocarbon carrier which can drip and quickly be spent and require recharging.

During manufacture of cylinder 1 an active ingredient (not shown) is incorporated into the wax mixture. It is this active ingredient that will act against the biting insects to give the animal some relief. Any suitable insecticide may be used. Different formulas may be used for different animals in different circumstances, as would be understood by the person skilled in the art. It should be noted that different jurisdictions have different rules on use of these active ingredients and some are not available for use, in some countries. However, some useful examples are described below. Clearly, these are generally illustrative examples only and any suitable additive could be used instead of an insecticide in other use of the invention.

Cylinder block 1 is illustrated as a permethrin cylinder block and the active ingredient permethrin, is mixed into the wax mix, with the total block 1700 millilitres and the volume of active ingredient 20 millilitres at concentration of 87 grams per litre. As illustrated in the volume data below for the inventive block of 1700 millilitres, with the active ingredient Permethrin, in addition to the 20 millilitres, active ingredient there is 66 millilitres of coconut coir, 830 millilitres of paraffin white oil, 200 millilitres of petroleum jelly, which is mixed with 584 millilitres of the wax blend as described above. The different mixtures, and proportions found to be useful for each active ingredient are as listed in the Volume Data table below.

Some example active ingredients and concentrations for use incorporated into cylinder block 1 as follows:

**TABLE a)**

| **No.** | **Active Ingredient:-** | **Product name:-** | **Concentration** |
|---|---|---|---|
| 1 | Permethrin | PERMETHRIN | 87g/ltr |
| 2 | Chlorfenvinphos | CHLORFENVINPHOS | 200g/ltr |
| 3 | Diazinon | DIAZINON | 200g/ltr |
| 4 | Phosmet | PHOSMET | 150g/ltr |
| 5 | Citronella, Eucalyptus | | Concentrate |

The following **TABLE 1** provides the suggested volume data for example product 1.7L block mixes as described in embodiment 1:-

| **Volume Data - per 1.7L block (active ingredient - Chlorfenvinphos)** | | | |
|---|---|---|---|
| **Item** | **Ingredient** | **Value** | **Unit** |
| 1 | Volume of Supona (Trade Mark) per treatment block (active ingredient - Chlorfenvinphos) | 150 | ml |
| 2 | Volume coconut fiber per block | 66 | ml |
| 3 | Total volume of paraffin white oil per 1.7L treatment block | 700 | ml |
| 4 | Total volume of petroleum jelly per 1.7L treatment block | 200 | ml |
| 5 | Total volume of wax per 1.7L treatment block | 584 | ml |

**TABLE 1 continued:**

| **Volume Data - per 1.7L block (active ingredient - Diazinon)** | | | |
|---|---|---|---|
| **Item** | **Ingredient** | **Value** | **Unit** |
| 1 | Volume of Diazinon per treatment block (active ingredient - Diazinon) | 150 | ml |
| 2 | Volume coconut fiber per block | 66 | ml |
| 3 | Total volume of paraffin white oil per 1.7L treatment block | 700 | ml |
| 4 | Total volume of petroleum jelly per 1.7L treatment block | 200 | ml |
| 5 | Total volume of wax per 1.7L treatment block | 584 | ml |

| **Volume Data - per 1.7L block (active ingredient - Permethrin)** | | | |
|---|---|---|---|
| **Item** | **Ingredient** | **Value** | **Unit** |
| 1 | Volume of Brute (Trade Mark) Permethrin per treatment block (active ingredient - Permethrin) | 20 | ml |
| 2 | Volume coconut fiber per block | 66 | ml |
| 3 | Total volume of paraffin white oil per 1.7L treatment block | 830 | ml |
| 4 | Total volume of petroleum jelly per 1.7L treatment block | 200 | ml |
| 5 | Total volume of wax per 1.7L treatment block | 584 | ml |

| **Volume Data - per 1.7L block (active ingredient - Phosmet)** | | | |
|---|---|---|---|
| **Item** | **Ingredient** | **Value** | **Unit** |
| 1 | Volume of Imidan (Trade Mark) Phosmet per treatment block (active ingredient - Phosmet) | 20 | ml |
| 2 | Volume coconut fiber per block | 66 | ml |
| 3 | Total volume of paraffin white oil per 1.7L treatment block | 830 | ml |
| 4 | Total volume of petroleum jelly per 1.7L treatment block | 200 | ml |
| 5 | Total volume of wax per 1.7L treatment block | 584 | ml |

| **Volume Data - per 1.7L block (active ingredient - Citronella & Eucalyptus essential oils)** | | | |
|---|---|---|---|
| **Item** | **Ingredient** | **Value** | **Unit** |
| 1 | Volume of Citronella oil per treatment block (active ingredient - Citronella oil) | 63 | ml |
| 2 | Volume of Eucalyptus oil per treatment block (active ingredient - Eucalyptus oil) | 37 | ml |
| 3 | Volume coconut fiber per block | 66 | ml |
| 4 | Total volume of paraffin white oil per 1.7L treatment block | 750 | ml |
| 5 | Total volume of petroleum jelly per 1.7L treatment block | 200 | ml |
| 6 | Total volume of wax per 1.7L treatment block | 584 | ml |

| **Volume Data - per 1.7L block (active ingredient - Essential Oils - Cattle Coat)** | | | |
|---|---|---|---|
| 1 | Volume of Cattle Coat (Trade Mark) per treatment block (active ingredients various) | 1000 | ml |
| 2 | Volume coconut fiber per block | 66 | ml |
| 3 | Total volume of petroleum jelly per 1.7L treatment block | 50 | ml |
| 4 | Total volume of wax per 1.7L treatment block | 584 | ml |

| **Volume Data - per 1.7L block (active ingredient - Essential Oils - Pony Coat)** | | | |
|---|---|---|---|
| 1 | Volume of Pony Coat (Trade Mark)per treatment block (active ingredient -various) | 1000 | ml |
| 2 | Volume coconut fiber per block | 66 | ml |
| 3 | Total volume of petroleum jelly per 1.7L treatment block | 50 | ml |
| 4 | Total volume of wax per 1.7L treatment block | 584 | ml |

The following **TABLE 2** provides the suggested volume data for example product mixes as recently developed over further research, for a 1.18L block (refer embodiment 3).

**TABLE 2:-**

| **Volume Data - per 1.18L block (active ingredient - Chlorfenvinphos)** | | | |
|---|---|---|---|
| **Item** | **Ingredient** | **Value** | **Unit** |
| 1 | Volume of Chlorfenvinphos per treatment block | 30 | ml |
| 2 | Volume coconut fiber per block | 35 | ml |
| 3 | Total volume of paraffin oil per treatment block | 596 | ml |
| 4 | Total volume of petroleum jelly per treatment block | 74 | ml |
| 5 | Total volume of microcrystaline wax per treatment block | 64 | ml |
| 6 | Total volume of SASOLWAX H1(Trade Mark) per treatment block | 381 | ml |

| **Volume Data - per 1.18L block (active ingredient - Diazinon)** | | | |
|---|---|---|---|
| **Item** | **Ingredient** | **Value** | **Unit** |
| 1 | Volume of diazinon per treatment block | 30 | ml |
| 2 | Volume coconut fiber per block | 35 | ml |
| 3 | Total volume of paraffin oil per treatment block | 596 | ml |
| 4 | Total volume of petroleum jelly per treatment block | 74 | ml |
| 5 | Total volume of microcrystaline wax per treatment block | 64 | ml |
| 6 | Total volume of SASOLWAX H1(Trade Mark) per treatment block | 381 | ml |

| **Volume Data - per 1.18L block (active ingredient - Phosmet)** | | | |
|---|---|---|---|
| **Item** | **Ingredient** | **Value** | **Unit** |
| 1 | Volume of Phosmet per treatment block | 30 | ml |
| 2 | Volume coconut fiber per block | 35 | ml |
| 3 | Total volume of paraffin oil per treatment block | 596 | ml |
| 4 | Total volume of petroleum jelly per treatment block | 74 | ml |
| 5 | Total volume of microcrystaline wax per treatment block | 64 | ml |
| 6 | Total volume of SASOLWAX H1(Trade Mark) per treatment block | 381 | ml |

| **Volume Data - per 1.18L block (active ingredient - Permethrin)** | | | |
|---|---|---|---|
| **Item** | **Ingredient** | **Value** | **Unit** |
| 1 | Volume of Permethrin per treatment block | 22 | ml |
| 2 | Volume coconut fiber per block | 35 | ml |
| 3 | Total volume of paraffin oil per treatment block | 604 | ml |
| 4 | Total volume of petroleum jelly per treatment block | 74 | ml |
| 5 | Total volume of microcrystaline wax per treatment block | 64 | ml |
| 6 | Total volume of SASOLWAX H1(Trade Mark) per treatment block | 381 | ml |

Table 2 is continued below:

**TABLE 2 Continued:**

| **Volume Data - per 1.18L block (Horse Care / Non-Chemical)** | | | |
|---|---|---|---|
| **Item** | **Ingredient** | **Value** | **Unit** |
| 1 | Volume of Eucalyptus Oil per treatment block | 17 | ml |
| 2 | Volume of Tea Tree Oil per treatment block | 2.5 | ml |
| 3 | Volume of Lemon Grass Oil or Lavender Oil per treatment block | 1 | ml |
| 4 | Volume of Lavender Oil per treatment block | 1 | ml |
| 5 | Volume of Aster Amellus Root Juice per treatment block | 0.5 | ml |
| 6 | Volume of Pine Oil per treatment block | 0.5 | ml |
| 7 | Volume of Citronella per treatment block | 2.5 | ml |
| 8 | Volume of Palmarosa oil per treatment block | 1 | ml |
| 9 | Volume of Clionasterol per treatment block | 4 | ml |
| 10 | Volume coconut fiber per block | 35 | ml |
| 11 | Total volume of paraffin oil per treatment block | 596 | ml |
| 12 | Total volume of petroleum jelly per treatment block | 74 | ml |
| 13 | Total volume of microcrystaline wax per treatment block | 64 | ml |
| 14 | Total volume of SASOLWAX H1(Trade Mark) per treatment block | 381 | Ml |

| **Volume Data - per 1.18L block (Cattle Care/Non-chemical)** | | | |
|---|---|---|---|
| **Item** | **Ingredient** | **Value** | **Unit** |
| 1 | Volume of Eucalyptus Oil per treatment block | 17 | ml |
| 2 | Volume of Tea Tree Oil per treatment block | 2.5 | ml |
| 3 | Volume of Lemon Grass or Lavender Oil per treatment block | 1 | ml |
| 7 | Volume of Citronella per treatment block | 9.5 | ml |
| 10 | Volume coconut fiber per block | 35 | ml |
| 11 | Total volume of paraffin oil per treatment block | 596 | ml |
| 12 | Total volume of petroleum jelly per treatment block | 74 | ml |
| 13 | Total volume of microcrystaline wax per treatment block | 64 | ml |
| 14 | Total volume of SASOLWAX H1(Trade Mark) per treatment block | 381 | ml |

| | | | |
|---|---|---|---|
| ** No allowance in the Table 2 volumes has been made for the centre wear bush if used, deduct according to centre bush size.* | | | |

Cylinder 1 as in the illustrative example is formulated with the mix as described in Table 1, including the active ingredient Permethrin.

Each of the illustrative examples including different active ingredients, or the same active ingredient and a different wax mix. However, each of the described mixtures could be used for cylinder 1 instead, including those of Table 2 with the adapted volumes and proportions. Clearly, there is significant scope for variance in the wax mix, ratios and the concentration and use of the active ingredients. The above are non-limiting illustrative examples. A UV inhibitor can be used with any of the mixes to act against the UV from the action of the sun in use as illustrated in the TABLES. The illustrated volume of the UV inhibitor is 0.005%.

The particular method or order of making the cylinder blocks can be varied as would be understood by the person skilled in the art. In the main part the ingredients can be introduced to a container, with contingency to allow for filling of the shaped pots. Once the mixture is thoroughly mixed the containers can be filled, and allowed to set, before being tipped out ready for use. Cylindrical conduit 16 is formed in the container to create the shape as illustrated in Figure 1. Other forms could use connectors, chains, or attachments at either side and omit the conduit. However, the simple method of use when including conduit 16 and the ability to use it on existing structures, such as for the prior art hanging apparatus, or on a pole or post, gives great versatility to this form of the invention.

Depending on the method of manufacture the wax mixture can be mixed by hand or by use of an automated system before being poured into moulds and set in the desired shape. Clearly, other shapes could be used but the cylinder block is a familiar shape and readily used on a chain or rope for cattle, for example. Any suitable shape could be used in other forms.

Referring to Figure 13, hanging apparatus 130 is shown as an illustrative example of how block 1 and other similar blocks (see below) may be used to treat animals. As illustrated existing posts 132, 134 as previously used for another purpose are repurposed for hanging apparatus 130. New posts may be used or any upright such as a tree could be used instead. Each of posts 132 and 134 has loop attachment 136 and 138, to which chain 140 is strung. The example uses a chain and attachments but could be readily replaced with a rope, or any suitable matter to hang the blocks. Chain 140 is shown threaded through cylindrical conduit 16 of two blocks 1 and of the equivalent of other different blocks 142 and 144. If the conduit is replaced by loops or other attachments these can connect together instead. These other blocks are illustrated as containing different active ingredients and mixes to show the strong variety and numerous options the invention provides. However, in general use all the blocks will be the same. The number of cylindrical blocks 1 is shown as 6, and 6 to 10 is a good example for use for treatment for cattle. Other numbers can be used and it is a further advantage of the invention that the number of blocks can be varied in this way. A particular farmer can replace some rather than all of the blocks if need be, or add further blocks.

The blocks can be seen to be slung at an appropriate height for cattle to rub themselves on the blocks. Generally, the blocks may be worn out together and so these may be replaced together. The cattle are looking to scratch and rid themselves of the biting flies and can do so on the slung blocks 1. As they rub their back, side or face, for example, onto the blocks the active ingredient with wax carrier is transferred in a controlled manner to the animal. The blocks gradually contact the cattle as they rub themselves and apply the active ingredient in wax carrier to kill the biting flies or insects. Each block 1 and its controlled release of active ingredient will last around 12 weeks in this form of the invention, in other forms 20 weeks or longer are possible. The length of time will depend on the number of animals that visit the block and the amount of use it gets, as well as the particular formula and format of the block in use. The blocks may be adapted to last a greater or lesser amount of time.

Each block can then be replaced as it becomes worn down or on a program of regular replacement, periodically. It is likely that the cattle will therefore be able to scratch and receive the controlled application of the insecticide active ingredient continuously, over the life of the block. The animals will be happier with this situation, more comfortable and less bothered and restless. The cattle that have less distress due to biting flies are most likely to present for sale with good weights and be generally in much better health.

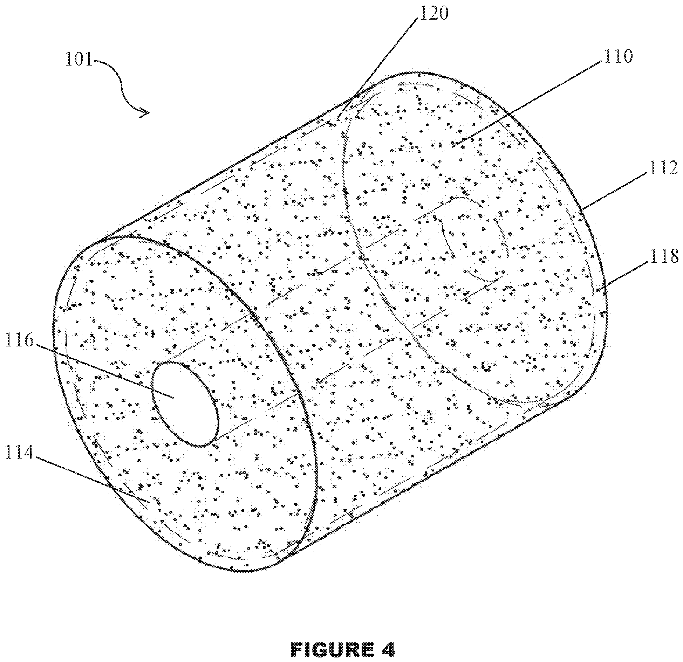

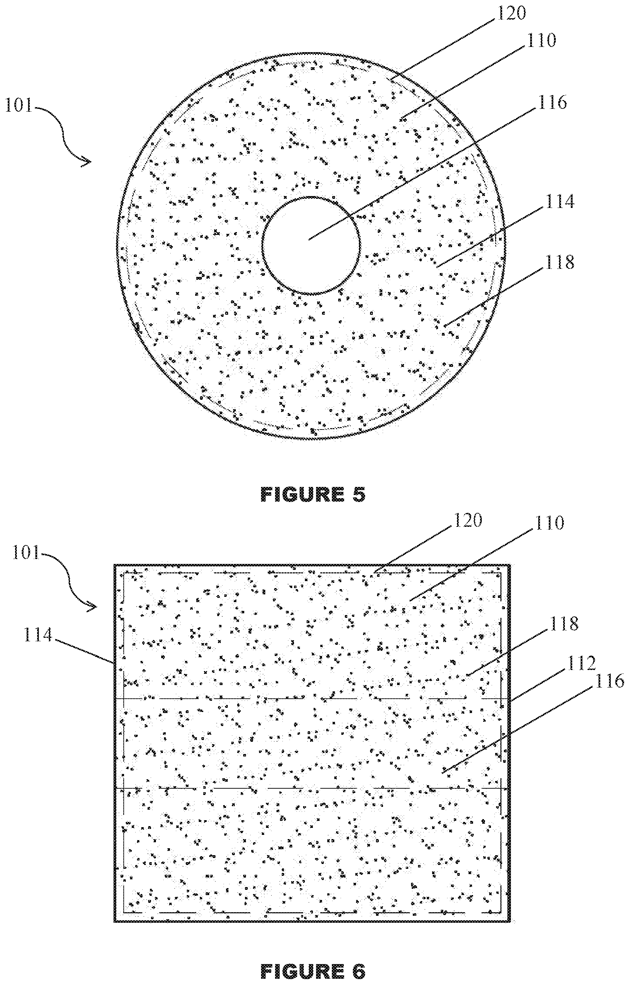

Referring now to Figures 4 to 6, a variant to the present inventive block is illustrated, similar to the first and with similar reference numerals used throughout. The difference from the first is that there is an external wax layer to provide a better aesthetic look before use, and also to provide some protection to the block until in use.

As illustrated block 101 has body 110, with ends 112 and 114, between which runs cylindrical conduit 116 and is a similar cylindrical shape to block 1. Fibre 118 is included, coconut coir fibres as described above, in the same wax mixture as described in Table 1 for the organic, essential oil form of the invention. Other wax mixtures or proportions may be substituted instead, and other active ingredients and percentages and proportions as set out in Table 1, Table 2 or elsewhere.

As illustrated each body 101 is 127 millimetres wide by 140 millimetres wide 134 millimetres as the first embodiment but surrounded by a 3 millimetre external wax layer. The external wax layer in the given example is to cover the coconut coir for sale presentation. Other out layer arrangements may be used instead. In all other ways body 101 is identical to body 1. A UV inhibitor is used, in the example, 2-Hydroxy-4-n-octoxybenzophenone incorporated into the wax to act against degradation due to sun exposure. Again the size is convenient but can be varied for other applications.

In use cylinder blocks 101 are used as block 1 hung or strung to create a rub area for the animals whereby they gain a controlled treatment.

Cylinder block 101 may use any of the active ingredients listed above at Table a), Table 1 or Table 2, or other suitable active ingredients. As shown the active ingredient includes concentrates of citronella 63 millilitres and 37 millilitres eucalyptus for a 1700 millilitre block, mixed into the wax carrier. In this form of the invention a user may be interested in avoiding using some of the more traditional chemicals for insecticide, and wish to use the natural essential oils of citronella and eucalyptus as an organic alternative. The mix and block of 1180 millimetres as described for Table 2 could be used instead. In which case the block will include a mix of Eucalyptus oil, tea tree oil, lemon grass oil, lavender oil, aster amellus root juice, pine oil, citronella, palmarosa oil and clionasterol as active ingredients in volumes as set out in Table 2. The mixes here described are of particular use for action against horse fly *Tabanide* and face fly *Musca Autumnalis.* In use with essential oils there is the added benefit that the pervading oils act as a deterrent to the flies and insects approaching the animal, as well as the action to treat the animal so that insects in contact with the treatment die. A deterrent essential oil or similar could be used in any of the forms of invention, as described above. Variation to the proportions may be made and some omitted in variants of the invention.

Block 101 can be made exactly as described for block 1 manually, poured into a suitable container of a suitable shape, and set and removed for use.

Although block 101 is not specifically illustrated in Figure 13 all of the blocks shown in that illustration could be readily replaced with block 101 instead. The method of use is as described for block 1 and the method of Figure 13.

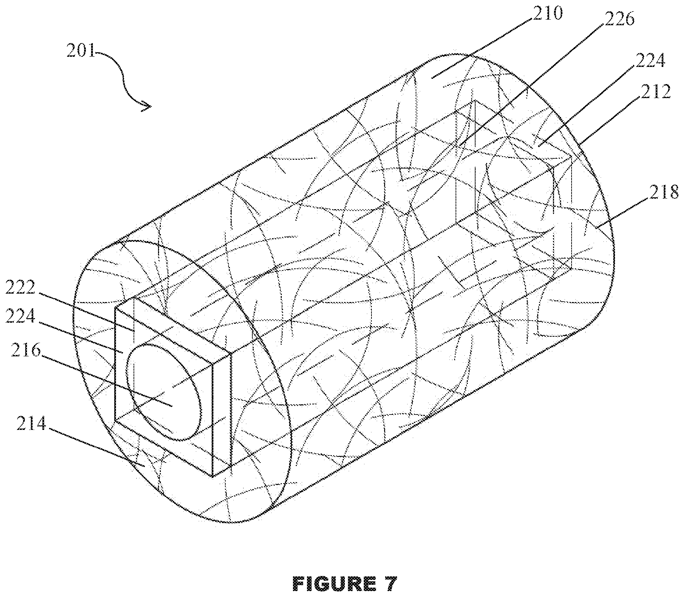

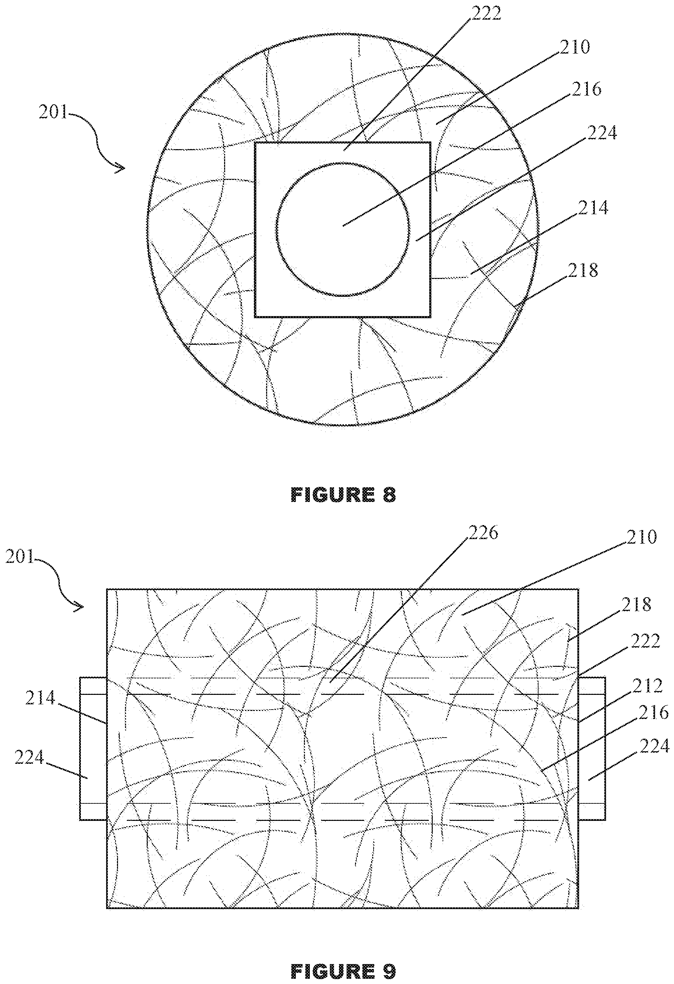

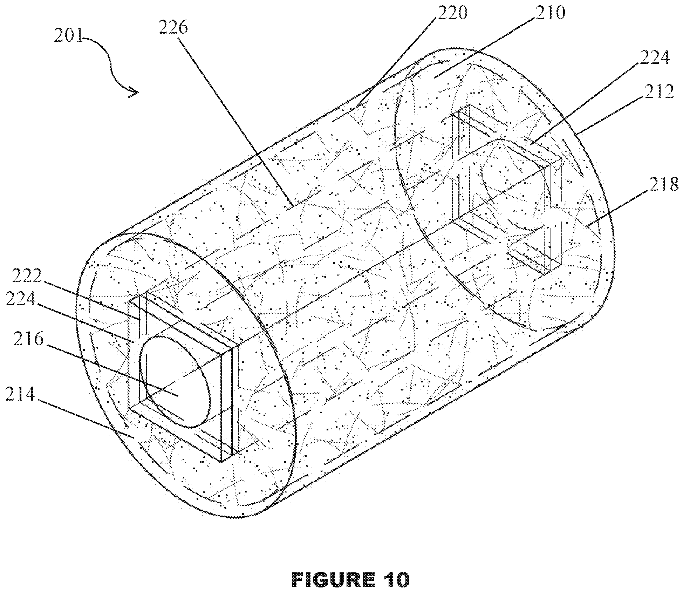

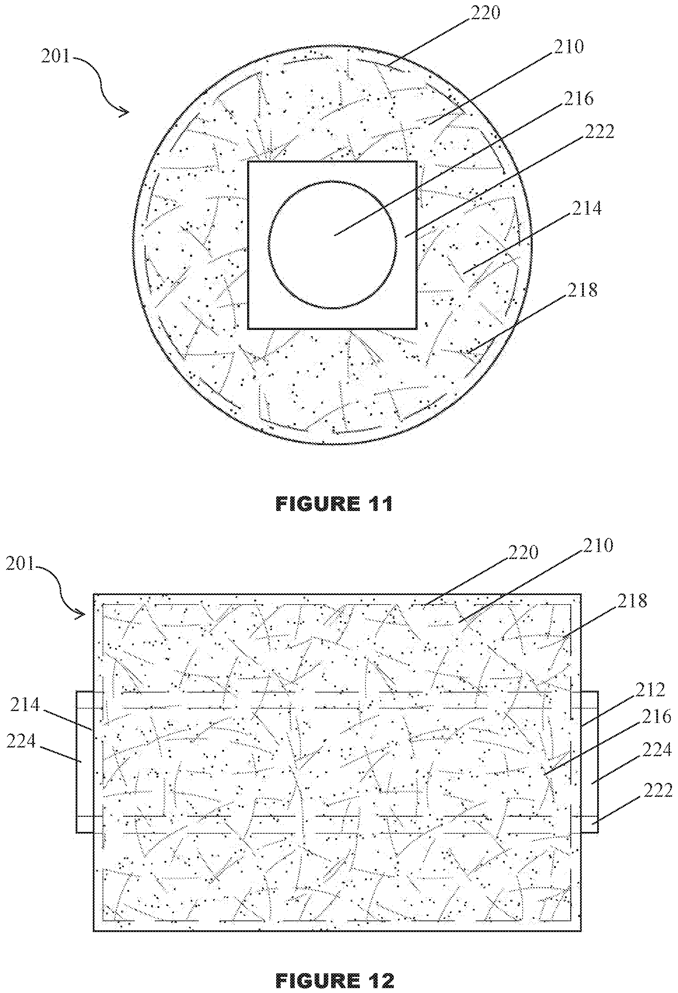

Referring now to Figures 7 to 12, an improved variant to the present inventive block is illustrated, similar to the second and again, with similar reference numerals used throughout. The method of mixing and moulding the blocks is the same as described elsewhere for the other forms of the invention. The difference of the described block from the first and second embodiments is the inclusion of a very useful biodegradable bush that assists to prolong the life of the block, and once used the material does not adversely impact the environment. The block of Figures 7 to 12 in any of its forms or variants can be used in place of block 1 as illustrated for Figure 13.

As illustrated block 201 has body 210, with ends 212 and 114, between which runs cylindrical conduit 216 as for the first and second embodiments, and is a similar cylindrical shape to block 1 and block 101. Fibres 218 are included, and again is a mix of coconut coir fibres in a wax mixture. The example of the third embodiment is a mix from Table 2, above in a 1180 millilitre block, with 22 millilitres of permethrin mixed into a wax mix containing, Sasolwax H1 (Trade Mark), 381 millilitres and microcrystalline wax of 64 millilitres, mixed with paraffin oil 604 millilitres and petroleum jelly 74 millilitres. Coconut coir fibres for the structural means of 35 millimetres, which equates to about 12 grams, per block. The third embodiment can be made according to any suitable wax and active ingredient mix of the invention, including any of those listed in Table 1 instead.

As illustrated each body 201 is 100 millimetres wide, by 145 millimetres long as including a 3 millimetre external wax layer 220 to protect the block until use, and for presentation. The central core 216, has a diameter 32 millimetres as illustrated with bush 222, the different feature for the third embodiment being 42 millimetres across with a length of 155 millimetres, ends 224 of bush 222 at either end of body 226, project 5 millimetres from each end of block 201. Each bush 222 is formed of timber as a strong, light material to protect the inner surface of wax body 210 from the mounting. Where the mounting is a chain when block 201 is mounted as illustrated in Figure 13 (for example in place of block 1) the action of the chain on the inner surface can quickly reduce the life of the block, and so it has been found beneficial to include a protective bush. Any suitable protective bush may be included, but it is most useful to have one that can be left in the environment and does not cause undue pollution, is biodegradable. Wood is particularly useful, but is just one example, bamboo products, or natural plastics or composite materials may be used instead. Ends 224 themselves protect against rubbing and action against ends 212 and 214 of block 201, giving overall protection against the mounting. Overall the block of the third embodiment illustrates a useful form of block that can be adapted to different active ingredients and different mountings, as required for the particular application.

Although not illustrated, a further useful example may be given where block is made with percentages as follows 32.2 percent synthetic high temperature wax, 5.4 percent microcrystalline wax, 6.2 percent petroleum jelly, 50.8 percent mineral oil with 2.9 percent coconut coir and a 2.5 percent of active ingredient diazinon, chlorfenvinphos or phosmet. The block made to the proportions and moulded for use as described elsewhere has been found to be useful for 12 or 20 weeks of use in the field to act against biting flies for cattle. If the biodegradable bush is included, this acts against where from the mounting, which again prolongs the useful life of the block. Other examples are apparent from the Table 1 and Table 2 in particular.

The inventor has developed a clever invention as defined in claim 1 that uses a wax cylinder as carrier for active ingredients to create a solid block. This solid block replaces the need to use liquid, uncontrolled carriers such as diesel or white oil which may drip or spill. The subject inventive block can provide controlled application of the active ingredient held in the wax carrier over a longer period of time and with less maintenance than the prior art. The cattle get better treatment against insects and the farmer saves time and money, with a better end result.

It will be apparent to a person skilled in the art that changes may be made to the embodiments disclosed herein without departing from the scope of the invention in its various aspects.

### REFERENCE SIGNS LIST:

| | **Embodiment 1:-** | | **Embodiment 3:-** |
|---|---|---|---|
| **1** | Cylinder block | **201** | Cylinder block |
| **10** | Body | **210** | Body |
| **12** | End | **212** | End |
| **14** | End | **214** | End |
| **16** | Cylindrical conduit | **216** | Cylindrical conduit |
| **18** | Fibres | **218** | Fibres |

| | **Embodiment 2:-** | **220** | Layer |
|---|---|---|---|
| **101** | Cylinder block | **222** | Bush |
| **110** | Body | **224** | Ends (2) of bush |
| **112** | End | **226** | Body of bush |
| **114** | End | | |
| **116** | Cylindrical conduit | | |
| **118** | Fibres | | |
| **120** | Layer | | |
| | | | |
| **130** | Hanging apparatus | | |
| **132** | Post | | |
| **134** | Post | | |
| **136** | Loop attachment | | |
| **138** | Loop attachment | | |
| **140** | Chain | | |
| **142** | Block | | |
| **144** | Block | | |
| | | | |

## Claims

1. An animal treatment block (1, 101, 201) for applying a treatment to an animal against the action of biting insects, the animal treatment block (1, 101, 201) including:
a body (10, 110, 210) including a carrier medium, wherein the carrier medium includes synthetic high temperature hard wax;
and further wherein structural means (18, 118, 218) including a plurality of fibres (18) are incorporated into the carrier medium of the body (10, 110, 210) to give structural integrity;
an active ingredient incorporated into the carrier medium of the body (10, 110, 210); and
wherein the animal treatment block (1, 101, 201) is a substantially cylindrical block (10, 110, 210),
wherein the body (10, 110, 210), including the carrier medium with incorporated active ingredient and structural means (18, 118, 218) , may be mounted such that an animal can readily contact the body (10, 110, 210), and is suitable to be used as a roll or rub to apply the treatment to the animal by rolling or rubbing of the cylinder block (10, 110, 210) against the skin of the animal, whereby the active ingredient is applied to the animal in a controlled manner, through application of the carrier medium, and further wherein, the block (1, 101, 201) is configured to provide treatment for animals for several weeks.

2. The animal treatment block (1, 101, 201) according to claim 1, wherein paraffin oil is included in the carrier medium.

3. The animal treatment block (1, 101, 201) of claim 1 or 2, wherein the synthetic wax is high temperature hard wax with a melting temperature of substantially 105 degrees Centigrade.

4. The animal treatment block (1, 101, 201) according to any one of claims 1 to 3, wherein a wax blend is included, suitable to use up to 35 degrees Centigrade.

5. The animal treatment block (1, 101, 201) according to any one of claims 1 to 4, wherein in the synthetic high temperature hard wax is used in the range of 5 to 50 percent.

6. The animal treatment block (1, 101, 201) according to any one of claims 1 to 5, wherein the active ingredient includes a blend of suitable essential oils chosen from the group: eucalyptus; tea tree; lemon grass; lavender; aster amellus root juice; pine oil, citronella; palmarosa; clionasteral; or similar.

7. The animal treatment block (1, 101, 201) according to any one of claims 1 to 5, wherein the active ingredient is chosen from the group: permethrin; chlorfenvinphos; coumaphos; diazinon; or phosmet, any suitable synthetic pyrethroid, any suitable organophosphate, or any suitable essential oil.

8. The animal treatment block (1, 101, 201) according to any one of claims 1 to 5, wherein the block (1, 101, 201) is 1180 millilitres, and the volume of active ingredient is 30 millilitres of the active ingredient chlorfenvinphos, diazinon, or phosmet.

9. The animal treatment block (1, 101, 201) according to any one of claims 1 to 5, wherein the block (1, 101, 201) is 1180 millilitres, and the volume of active ingredient is 22 millilitres of the active ingredient permethrin.

10. The animal treatment block (1, 101, 201) according to any one of claims 1 to 5, wherein the block (1, 101, 201) is 1180 millilitres, and the volume of active ingredient includes 2.5 millilitres of citronella oil and/or 2.5 millilitres of tea tree oil and/or 17 millilitres of eucalyptus oil or the block is 1180 millilitres, and the volume of active ingredient is 2.5 millilitres of citronella oil and 17 millilitres of eucalyptus oil with one or more of 2.5 millilitres of tea tree oil, 1 millilitres of lemon grass oil, 1 or 2 millimetres of lavender oil, 0.5 millilitres of aster amellus root juice, 0.5 millilitres of pine oil, 1 millilitres of palmarosa oil, and 4 millilitres of clionasterol.

11. The animal treatment block (1, 101, 201) according to any one of claims 1 to 10, wherein the fibres (18, 118, 218) are coconut coir.

12. The animal treatment block (1, 101, 201) of claim 11, wherein the coconut coir fibres (18, 118, 218) are substantially 150 to 200 millimetres in length.

13. The animal treatment block (1, 101, 201) according to claim 11 or 12, wherein the coconut coir fibres (18, 118, 218) are mixed in at a percentage of substantially 2.9 or 4 percent.

14. The animal treatment block (1, 101, 201) according to any one of claims 1 to 13, wherein the block is adapted to be useful to provide treatment for animals for up to 20 weeks.

## Patentansprüche

1. Tierbehandlungsblock (1, 101, 201) zum Auftragen einer Behandlung auf ein Tier gegen die Wirkung von stechenden Insekten, wobei der Tierbehandlungsblock (1, 101, 201) beinhaltet:
einen Körper (10, 110, 210), der ein Trägermedium beinhaltet, wobei das Trägermedium synthetisches Hochtemperatur-Hartwachs beinhaltet;
und wobei ferner strukturelle Mittel (18, 118, 218), die eine Vielzahl von Fasern (18) beinhalten, in das Trägermedium des Körpers (10, 110, 210) eingearbeitet sind, um strukturelle Integrität zu verleihen;
ein aktiver Wirkstoff, der in das Trägermedium des Körpers (10, 110, 210) eingearbeitet ist; und
wobei der Tierbehandlungsblock (1, 101, 201) ein im Wesentlichen zylindrischer Block (10, 110, 210) ist,
wobei der Körper (10, 110, 210), der das Trägermedium mit eingearbeitetem Wirkstoff und strukturellen Mitteln (18, 118, 218) beinhaltet, derart montiert werden kann, dass ein Tier ohne Weiteres den Körper (10, 110, 210) berühren kann, und der dazu geeignet ist, als eine Rolle oder Reibe verwendet zu werden, um die Behandlung auf dem Tier durch Rollen oder Reiben des Zylinderblocks (10, 110, 210) gegen die Haut des Tiers aufzutragen, wodurch der Wirkstoff auf dem Tier in einer kontrollierten Weise durch Auftragen des Trägermediums aufgetragen wird, und wobei ferner der Block (1, 101, 201) dazu konfiguriert ist, Behandlung für Tiere für mehrere Wochen bereitzustellen.

2. Tierbehandlungsblock (1, 101, 201) nach Anspruch 1, wobei Paraffinöl in dem Trägermedium enthalten ist.

3. Tierbehandlungsblock (1, 101, 201) nach Anspruch 1 oder 2, wobei das synthetische Wachs Hochtemperatur-Hartwachs mit einer Schmelztemperatur von im Wesentlichen 105 Grad Celsius ist.

4. Tierbehandlungsblock (1, 101, 201) nach einem der Ansprüche 1 bis 3,
wobei eine Wachsmischung enthalten ist, die zur Verwendung bis zu 35 Grad Celsius geeignet ist.

5. Tierbehandlungsblock (1, 101, 201) nach einem der Ansprüche 1 bis 4,
wobei das synthetische Hochtemperatur-Hartwachs in dem Bereich von 5 bis 50 % verwendet wird.

6. Tierbehandlungsblock (1, 101, 201) nach einem der Ansprüche 1 bis 5,
wobei der Wirkstoff eine Mischung aus geeigneten ätherischen Ölen beinhaltet, die ausgewählt sind aus der Gruppe: Eukalyptus; Teebaum; Zitronengras; Lavendel; Aster-Amellus-Wurzelsaft; Kiefernöl, Citronella; Palmarosa; Clionasteral; oder Ähnliches.

7. Tierbehandlungsblock (1, 101, 201) nach einem der Ansprüche 1 bis 5,
wobei der Wirkstoff ausgewählt ist aus der Gruppe: Permethrin; Chlorfenvinphos; Coumaphos; Diazinon; oder Phosmet, jedem geeigneten synthetischen Pyrethroid, jedem geeigneten Organophosphat oder jedem geeigneten ätherischen Öl.

8. Tierbehandlungsblock (1, 101, 201) nach einem der Ansprüche 1 bis 5,
wobei der Block (1, 101, 201) 1180 Milliliter beträgt und das Volumen an aktivem Wirkstoff 30 Milliliter des aktiven Wirkstoffs Chlorfenvinphos, Diazinon oder Phosmet beträgt.

9. Tierbehandlungsblock (1, 101, 201) nach einem der Ansprüche 1 bis 5,
wobei der Block (1, 101, 201) 1180 Milliliter beträgt und das Volumen an aktivem Wirkstoff 22 Milliliter des aktiven Wirkstoffs Permethrin beträgt.

10. Tierbehandlungsblock (1, 101, 201) nach einem der Ansprüche 1 bis 5,
wobei der Block (1, 101, 201) 1180 Milliliter beträgt, und das Volumen an aktivem Wirkstoff 2,5 Milliliter Citronellaöl und/oder 2,5 Milliliter Teebaumöl und/oder 17 mm und Eukalyptusöl beträgt, oder der Block 1180 Milliliter beträgt, und das Volumen an aktivem Wirkstoff 2,5 Milliliter Citronella und 17 Milliliter Eukalyptusöl mit einem oder mehreren von 2,5 Milliliter Teebaumöl, 1 Milliliter Zitronengrasöl, 1 oder 2 Milliliter Lavendelöl, 0,5 Milliliter Aster-Amellus-Wurzelsaft, 0,5 mm Kiefernöl, 1 Milliliter Palmarosaöl und 4 Milliliter Clionasterol beträgt.

11. Tierbehandlungsblock (1, 101, 201) nach einem der Ansprüche 1 bis 10,
wobei die Fasern (18, 118, 218) Kokosfasern sind.

12. Tierbehandlungsblock (1, 101, 201) nach Anspruch 11, wobei die Kokosfasern (18, 118, 218) im Wesentlichen 150 bis 200 Millimeter lang sind.

13. Tierbehandlungsblock (1, 101, 201) nach Anspruch 11 oder 12,
wobei die Kokosfasern (18, 118, 218) in einem Prozentsatz von im Wesentlichen 2,9 oder 4 Prozent beigemischt sind.

14. Tierbehandlungsblock (1, 101, 201) nach einem der Ansprüche 1 bis 13,
wobei der Block dazu angepasst ist, zum Bereitstellen von Behandlung für Tiere für bis zu 20 Wochen nutzbar zu sein.

## Revendications

1. Bloc (1, 101, 201) de traitement pour animaux permettant d'appliquer un traitement à un animal contre l'action d'insectes piqueurs, le bloc (1, 101, 201) de traitement pour animaux incluant :
un corps (10, 110, 210) incluant un milieu porteur, dans lequel le milieu porteur inclut une cire dure synthétique à haute température ;
et en outre dans lequel des moyens structurels (18, 118, 218) incluant une pluralité de fibres (18) sont incorporés dans le milieu porteur du corps (10, 110, 210) pour donner une intégrité structurelle ;
un ingrédient actif incorporé dans le milieu porteur du corps (10, 110, 210) ; et
dans lequel le bloc (1, 101, 201) de traitement pour animaux étant un bloc (10, 110, 210) sensiblement cylindrique,
dans lequel le corps (10, 110, 210), incluant le milieu porteur avec ingrédient actif et moyens structurels (18, 118, 218) incorporés, peut être monté de telle sorte qu'un animal peut facilement entrer en contact avec le corps (10, 110, 210), et est convenable pour être utilisé comme rouleau ou bande de frottement pour appliquer le traitement à l'animal par roulement ou frottement du bloc (10, 110, 210) cylindrique contre la peau de l'animal, moyennant quoi l'ingrédient actif est appliqué à l'animal de manière contrôlée, par l'application du milieu porteur, et en outre, le bloc (1, 101, 201) étant configuré pour fournir un traitement à des animaux pendant plusieurs semaines.

2. Bloc (1, 101, 201) de traitement pour animaux selon la revendication 1, dans lequel
une huile de paraffine est incluse dans le milieu porteur.

3. Bloc (1, 101, 201) de traitement pour animaux selon la revendication 1 ou 2, dans lequel
la cire synthétique est une cire dure à haute température avec une température de fusion de sensiblement 105 degrés centigrades.

4. Bloc (1, 101, 201) de traitement pour animaux selon l'une quelconque des revendications 1 à 3, dans lequel
un mélange de cires est inclus, convenant à une utilisation jusqu'à 35 degrés centigrades.

5. Bloc (1, 101, 201) de traitement pour animaux selon l'une quelconque des revendications 1 à 4,
dans lequel, dans la cire dure synthétique à haute température est utilisée dans la plage de 5 à 50 pour cent.

6. Bloc (1, 101, 201) de traitement pour animaux selon l'une quelconque des revendications 1 à 5,
dans lequel l'ingrédient actif inclut un mélange d'huiles essentielles convenables choisies dans le groupe : eucalyptus ; arbre à thé ; Cymbopogon citratus; lavande ; jus de racine d'Aster amelle ; huile de pin, citronnelle ; palmarosa ; clionasteral ; ou similaire.

7. Bloc (1, 101, 201) de traitement pour animaux selon l'une quelconque des revendications 1 à 5,
dans lequel l'ingrédient actif est choisi dans le groupe : perméthrine ; chlorfenvinphos ; coumaphos ; diazinon ; ou phosmet, tout pyréthroïde synthétique convenable, tout organophosphate convenable, ou toute huile essentielle convenable.

8. Bloc (1, 101, 201) de traitement pour animaux selon l'une quelconque des revendications 1 à **5,**
le bloc (1, 101, 201) représentant 1180 millilitres, et le volume d'ingrédient actif représente 30 millilitres de l'ingrédient actif chlorfenvinphos, diazinon, ou phosmet.

9. Bloc (1, 101, 201) de traitement pour animaux selon l'une quelconque des revendications 1 à 5,
le bloc (1, 101, 201) représentant 1180 millilitres, et le volume d'ingrédient actif représente 22 millilitres de l'ingrédient actif perméthrine.

10. Bloc (1, 101, 201) de traitement pour animaux selon l'une quelconque des revendications 1 à 5,
le bloc (1, 101, 201) représentant 1180 millilitres, et le volume d'ingrédient actif inclut 2,5 millilitres d'huile de citronnelle et/ou 2,5 millilitres d'huile d'arbre à thé et/ou 17 millilitres d'huile d'eucalyptus ou le bloc représentant 1180 millilitres, et le volume d'ingrédient actif représente 2,5 millilitres d'huile de citronnelle et 17 millilitres d'huile d'eucalyptus avec un ou plusieurs parmi 2,5 millilitres d'huile d'arbre à thé, 1 millilitre d'huile de Cymbopogon citratus, 1 ou 2 millimètres d'huile de lavande, 0,5 millilitre de jus de racine d'Aster amelle, 0,5 millilitre d'huile de pin, 1 millilitre d'huile de palmarosa, et 4 millilitres de clionastérol.

11. Bloc (1, 101, 201) de traitement pour animaux selon l'une quelconque des revendications 1 à 10, dans lequel les fibres (18, 118, 218) sont du coco.

12. Bloc (1, 101, 201) de traitement pour animaux selon la revendication 11, dans lequel les fibres (18, 118, 218) de coco ont sensiblement une longueur de 150 à 200 millimètres.

13. Bloc (1, 101, 201) de traitement pour animaux selon la revendication 11 ou 12, dans lequel les fibres (18, 118, 218) de coco sont mélangées à un pourcentage sensiblement égal à 2,9 ou 4 pour cent.

14. Bloc (1, 101, 201) de traitement pour animaux **selon l'une quelconque des revendications 1 à 13,**
le bloc étant adapté pour être utile dans le traitement des animaux pendant une durée allant **jusqu'à** 20 semaines.
